(19) Europäisches Patentamt / European Patent Office / Office européen des brevets

(11) **EP 4 264 189 B1**

(12) **EUROPEAN PATENT SPECIFICATION**

(45) Date of publication and mention
of the grant of the patent:
**07.05.2025 Bulletin 2025/19**

(21) Application number: **21839622.4**

(22) Date of filing: **17.12.2021**

(51) International Patent Classification (IPC):
**G01D 5/353** (2006.01)    **G01D 18/00** (2006.01)
**A61B 1/00** (2006.01)    **G01K 11/3213** (2021.01)
**G01M 11/02** (2006.01)    **G01L 1/24** (2006.01)
**G01K 11/3206** (2021.01)    **G01B 11/16** (2006.01)
**G02B 6/028** (2006.01)    **A61B 5/00** (2006.01)
**G01M 11/00** (2006.01)

(52) Cooperative Patent Classification (CPC):
**G01D 5/35316; A61B 1/0017; G01D 5/35367;**
**G01D 18/00; G01M 11/31;** A61B 5/0042;
A61B 5/0071; A61B 5/0084; A61B 5/6847;
A61B 2562/0233; G01B 11/18; G01K 11/3206;
G01K 11/3213

(86) International application number:
**PCT/GB2021/053369**

(87) International publication number:
**WO 2022/129950 (23.06.2022 Gazette 2022/25)**

(54) **OPTICAL TRANSFORM CHARACTERISATION**

CHARAKTERISIERUNG EINER OPTISCHEN TRANSFORMATION

CARACTÉRISATION DE LA TRANSFORMATION OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2020 GB 202020197**

(43) Date of publication of application:
**25.10.2023 Bulletin 2023/43**

(73) Proprietor: **Oxford University Innovation Limited Botley, Oxfordshire OX2 0JB (GB)**

(72) Inventors:
• **BOOTH, Martin J.**
**Botley Oxfordshire OX2 0JB (GB)**
• **TURCOTTE, Raphael**
**Botley Oxfordshire OX2 0JB (GB)**
• **SALTER, Patrick S.**
**Botley Oxfordshire OX2 0JB (GB)**
• **FELLS, Julian**
**Botley Oxfordshire OX2 0JB (GB)**

(74) Representative: **Barker Brettell LLP**
**100 Hagley Road**
**Edgbaston**
**Birmingham B16 8QQ (GB)**

(56) References cited:
WO-A1-2020/095071    WO-A2-2013/144898
US-B2- 10 254 534

**Description**

TECHNICAL FIELD

The present invention relates to the characterisation of an optical transform, and to devices that employ optical transforms. More particularly, the invention relates to optical transforms resulting from transmission through an optical fibre, and to devices configured to employ optical transforms that correct for transmission through an optical fibre.

BACKGROUND

Multimode optical fibres (MMF) have a core diameter that is much larger that the wavelength of light that it the fibre is configured to carry, and can thereby support more than one mode of light propagation. MMF have a wide range of biomedical and industrial applications. Their small total diameter (typically 60-150 microns) provide access to hard-to-access locations and permits their insertion in biological tissue with limited disruption of physiological structures and functions. For example, minimally invasive micro-endoscopes based on MMF have been developed for in vivo brain imaging in the context of neuroscience studies. Such systems might be adapted for surgical guidance in other clinical applications (e.g. ear nose and throat applications).

A range of MMF applications, including the micro-endoscopes mentioned above, require spatially resolving optical signals for imaging or sensing. Contrast mechanisms for the optical signals in these applications include scattering and fluorescence, both in linear and nonlinear regimes, but could also be based on polarisation. Imaging with MMF can be implemented with widefield and point-scanning illumination and has been demonstrated with many modalities, for example: one-photon and multiphoton point-scanning fluorescence, confocal reflectance, coherent anti-Stokes Raman scattering, light-sheet, and SOFI (Super Resolution Optical Fluctuation Imaging. MMF are also used for microfabrication.

The location of the light source for illumination defines the proximal location and the position of the proximal end of the fibre. The proximal location refers to the space where the light source is located, and the proximal end comprises the MMF facet closest to the light source into which the illumination light is coupled. The distal location refers to the space at the other end of the fibre where the optical signal for imaging or sensing is generated, and the distal facet is the MMF facet at the distal end of the optical fibre that collects the optical signal. Between the proximal and distal end is the MMF, which serves as a waveguide to carry the illumination from the proximal end to the distal end and the optical signal from the distal end to the proximal end, which is optically coupled to a detector. The distal space (adjacent to the distal end) may be required to be free of optical elements (e.g. in a case where minimal invasiveness is required).

Optical effects occurring inside the MMF upon propagation cause the optical field to be transformed in an unpredictable, yet deterministic manner. This complex transformation T must be known for homomorphic mapping of information between the distal and proximal space with high spatial resolution as well as for spatially defined sensing in the distal space. Using a vectorial notation, **T,** known as the transmission matrix (TM), expresses the relationship between an input field at the proximal facet **x** and the resulting output field at the distal facet **y'** and, reciprocally through time-reversal, an input field **y** at a distal facet and the resulting output field at the proximal facet **x':**

$$y' = T_\lambda x \qquad (1)$$

$$y = T_\lambda^T x' \qquad (2)$$

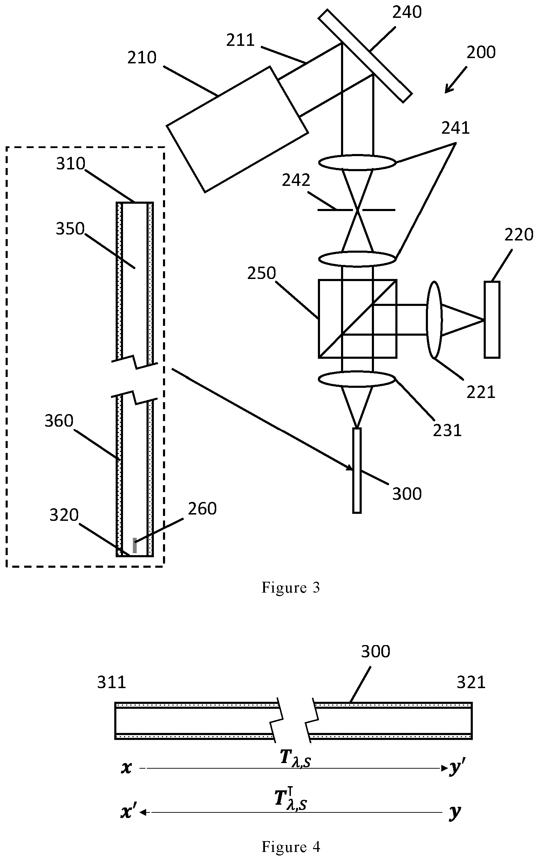

where the superscript $T$ indicates the transpose operation and the wavelength dependence of **T** has been made explicit. A definition for **T** may be valid for a narrow spectral bandwidth. This reciprocity of the transmission matrix is illustrated schematically in Figure 4.

The TM of an MMF is not only dependent on the illumination wavelength but also on the temperature and mechanical state of the fibre. This dependence on the fibre state S can be made explicit:

$$y' = T_{\lambda,S} x \qquad (3)$$

$$y = T_{\lambda,S}^T x' \qquad (4)$$

In several dynamic systems, the MMF may be expected to be continually deformed, in ways that cannot necessarily be predicted. In such cases, the problem of determining the TM for each state is not tractable.

In most practical scenarios, a TM can be evaluated with the fibre in an initial state $S_0$ before its positioning (e.g.

insertion in biological tissue). This approach can substantially simplify the evaluation of the TM upon continuous state changes (e.g. continuous deformation) using only proximal optical components, as the correction required to update the TM should also be continuous and small if evaluated in sufficiently close subsequent states. A correction matrix $C_{\lambda, S_{i-1} \to S_i}$ may be introduced to express the relationship between the TM of the current state $S_i$ to the previous state $S_{i-1}$:

$$T_{\lambda, S_i} = C_{\lambda, S_{i-1} \to S_i} T_{\lambda, S_{i-1}} \qquad (5)$$

where $i \in \mathbb{N}$.

Existing methods

Several methods have been proposed for evaluating the TM. Methods that require access to the distal end include:

  i) Direct interferometeric evaluation[1]
  ii) Phase conjugation[2]
  iii) Holographic optimisation[3]

[1] Čižmár, Tomáš, and Kishan Dholakia. "Shaping the light transmission through a multimode optical fibre: complex transformation analysis and applications in biophotonics." Optics Express 19.20 (2011): 18871-18884.
[2] Papadopoulos, Ioannis N., et al. "Focusing and scanning light through a multimode optical fiber using digital phase conjugation." Optics express 20.10 (2012): 10583-10590.
[3] Di Leonardo, Roberto, and Silvio Bianchi. "Hologram transmission through multi-mode optical fibers." Optics express 19.1 (2011): 247-254.

Methods have also been proposed that do not require a camera or light source at the distal end of the MMF, including:

  a) *Plöschner et al*[4]
  b) *Farahi et al*[5]
  c) *Gu et al*[6]
  d) *Chen et al*[7]
  e) *Gordon et al*[8]
  f) *Li et al*[9]

[4] Plöschner, M., Tyc, T. & Ćižmár, T. Seeing through chaos in multimode fibres. *Nature Photonics* **9**, 529-535 (2015).
[5] Farahi, S., Ziegler, D., Papadopoulos, I. N., Psaltis, D. & Moser, C. Dynamic bending compensation while focusing through a multimode fiber. Optics Express 21, 22504-22514 (2013).
[6] Gu, R. Y., Mahalati, R. N. & Kahn, J. M. Design of flexible multi-mode fiber endoscope. Optics Express 23, 26905-26918 (2015).
[7] Chen, H., Fontaine, N. K., Ryf, R., Neilson, D. T. & Winzer, P. Remote Spatio-Temporal Focusing over Multimode Fiber Enabled by Single-Ended Channel Estimation. Ieee Journal of Selected Topics in Quantum Electronics 1-1 (2020) doi:10.1109/jstqe.2020.2979241.
[8] Gordon, G. S. D. et al. Characterizing Optical Fiber Transmission Matrices Using Metasurface Reflector Stacks for Lensless Imaging without Distal Access. Phys Rev X 9, 041050 (2019).
[9] Li, S., Horsley, S. A. R., Tyc, T., Cizmar, T. & Phillips, D. B. Guide-star assisted imaging through multimode optical fibres. Arxiv (2020).

With the exception of *Plöschner,* all the other strategies require optical modulation beyond the distal facet. *Plöschner* requires that the TM, deformation and physical state of the MMF should be known, and allows a correction $C_{\lambda, S_{i-1} \to S_i}$ to be determined. In *Ploschner,* a theoretical approach is used to determine a TM for a bent fibre, based on prior knowledge of the shape of the bent fibre. *Plöschner* therefore is of limited practicality in contexts where the deformation of the fibre is not known or predictable. Furthermore, while this approach works well for a short fibre (<10cm) (which is fine for rodent applications) it is limited in how well it can deal with longer fibres with more complex conformation (including compound curvature). A 10cm fibre length is not sufficient for many clinical and industrial applications. The calculations are also complex.

In *Farahi* a reference light is delivered to the distal end of the MMF using a single mode fibre that runs in parallel with the multimode fibre (either concentrically, in a double clad fibre, or via a coupling prism in the case of a laterally offset single mode fibre). The reference light is diffracted by a holographic pattern recorded in a polymer layer at the distal end of the multimode fibre and reflected back into the multimode fibre. The diffracted reference light reflected back into the multimode fibre approximates an object point source, or virtual beacon. The proximal intensity distribution of this virtual beacon (after transmission through the multimode fibre) is measured, and correlated to a data bank of patterns corresponding to different fibre conformations. The data bank also includes the TM associated with each fibre conformation. This enables the TM for the fibre conformation to be determined from the detected virtual beacon at the proximal end of the fibre.

In *Gu* a thin reflective surface with a checkboard pattern is placed at the distal end of the MMF. Each square of the checkerboard pattern has a different reflection coefficient, with the result that spatial modulation of the amplitude of the reflectance signal is achieved. Light reflected from the checkerboard pattern is imaged at the proximal end of the fibre by a camera, and the resulting pattern used to determine an updated TM from an initial TM (that may be predetermined with access to both ends of the fibre, using and if the methods i) ii) or iii) mentioned above).

In *Chen* many single mode fibres are placed at the distal end, and the approach is somewhat equivalent to *Gu*. This is not practical in biomedical applications and other industrial applications, where it is not possible to place a lot of optical equipment at the distal end of the MMF.

In *Gordon* an extension of the method proposed by *Gu* is described, in which thin reflectors are stacked on the distal end of the MMF, and further encoding is done in each layer based on polarisation and wavelength. This is a more general approach, as it extends to non-unitary MMF. The approach is essentially the same as *Gu,* but instead of requiring N references, corresponding to the number of elements of the TM diagonal, NxN references are needed, which enables determination of the full TM.

*Farahi, Gu,* and *Gordon* all require assembly of micro-optics - which involves multiple complex components at the distal end (e.g. coupled to the distal facet of the MMF). This is complex and not-straightforward.

*Li* discloses the use of a guide star, which could be a fluorescent bead attached to the tip of the fibre. The TM may be found by an optimisation algorithm that determines an approximate transmission matrix by optimising constructive interference onto the guide star. The TM determined using this method is valid over an isoplanatic patch, the size of which is determined by the number of propagation invariant modes that have power at the position of the guide star. In practice, a guide star at the interface between the core and cladding was found to be optimal.

Each of the existing methods for compensating for the transfer function of a MMF is complex in practical implementation. Methods and devices that make it easier to determine a MMF TM are desirable, particularly if they facilitate determining corrections for movement/deformation of the MMF.

US 10 254 534 B2 discloses a system comprising: a light source; a spatial light modulator that phase shifts light from the light source into a first plurality of spatially independent modes; a beam splitter that splits light from the spatial light modulator into a first light beam and a second light beam; a first imager that images the first light beam; a multimode fiber coupler that couples a multimode fiber with the system; a second imager that images light from a distal end of the multimode fiber when coupled with the multimode fiber coupler for each of a second plurality of spatially independent modes; and a processor coupled with the spatial light modulator, the first imager and the second imager that creates a transmission matrix from the light received at the first imager and the second imager.

WO 2020/095071 discloses a method of characterizing an optical system, wherein the optical system comprises an optical fibre having a proximal end and a distal end, wherein the optical fibre comprises a non-single- mode optical fibre, and comprising a reflector assembly comprising a stack of reflectors disposed at the distal end of the optical fibre, wherein the stack of reflectors is arranged to provide different reflector matrices in dependence on illumination wavelength. The method comprises projecting calibration patterns at a plurality of characterization wavelengths onto the proximal end, then obtaining, at the proximal end, data relating to reflected calibration patterns. An instantaneous transmission matrix of the optical fibre is then determined using the data relating to the reflected calibration patterns and the reflector matrices.

WO 2013/144898 discloses a multimode waveguide illuminator and imager relying on a wave front shaping system that acts to compensate for modal scrambling and light dispersion by the multimode waveguide. A first step consists of calibrating the multimode waveguide and a second step consists in projecting a specific pattern on the waveguide proximal end in order to produce the desired light pattern at its distal end. The illumination pattern can be scanned or changed dynamically only by changing the phase pattern projected at the proximal end of the waveguide. The third and last step consists in collecting the optical information, generated by the sample, through the same waveguide in order to form an image.

SUMMARY

According to the invention in claim 1, there is provided a method of determining an optical transform imparted by a multimode optical fibre, wherein the multimode fibre comprises a proximal end, a distal end, and at least one modified region between the proximal end and distal end, the modified region configured to transmit light toward the proximal end in response to light propagating through the multimode optical fibre from the proximal end to the distal end, the method comprising:

coupling forward propagating light into the proximal end of the multimode optical fibre;

detecting, at the proximal end, backward propagating light transmitted from the at least one modified region in response to the forward propagating light;

determining an optical transform from the detected backward propagating light.

Determining the optical transform may comprise:

i) determining a transmission matrix of the multimode optical fibre, defining a relationship between an input field at a proximal facet and the resulting output field at a distal facet; and/or

ii) determining a correction matrix for correcting a predetermined transmission matrix (for example to correct changes in conformation of the optical fibre).

The transmission matrix comprises a matrix of complex coefficients relating the input field amplitude and phase of each of a plurality of input modes to the output field amplitude and phase of each of a plurality of output modes. The correction matrix may comprise a set of corrections for correcting a transmission matrix (e.g. as defined in equation (5)).

Providing a modified region in the MMF is a more elegant solution than prior art methods that involve optics appended to the distal facet of the MMF, or that rely on optical components arranged about the distal end of the MMF.

The at least one modified region may comprise at least one fibre Bragg grating. A fibre Bragg grating can be written in a MMF, for example using laser micromachining. The at least one modified region may comprise at least one fluorescent colour centre. A fluorescent colour centre will emit light at a different wavelength to the illuminating light, which may make it possible to perform sensing using the MMF at the same time as determining the optical transform of the MMF (and using the optical transform to optimise/correct the sensing).

The at least one modified region may comprise one or more modified regions disposed in the fibre core and/or fibre cladding.

There may be a plurality of modified regions, with at least some of the modified regions at different lateral positions and/or at different longitudinal positions between the proximal and distal end.

The at least one modified region may comprise a plurality of fibre Bragg gratings, and at least some of the fibre Bragg gratings may have different: period, reflectivity and/or orientation/polarisation.

The at least one modified region may comprise one or more chirped fibre Bragg gratings.

The forward propagating light may be a first forward propagating light field and the backward propagating light may be a first backward propagating light field, and the method may further comprise:

transmitting a second forward propagating light field for sensing/imaging a scene adjacent to the distal end;

detecting a second backward propagating light field transmitted through the fibre resulting from the second forward propagating light field, the method further propagating light field to correct/optimise an image formed using the second backward propagating light field.

Correcting/optimising the image may comprise controlling an active optical element to modify the second forward propagating light field.

The active optical element may comprise a spatial light modulator configured to modify the spatial distribution of phase of the second forward propagating light field

The method may comprise performing point scanning microscopy using a plurality of second forward propagating light fields.

Correcting/optimising the image may comprise computationally reconstructing an image from the results of detecting the second backward propagating light field.

The first forward propagating light field and the second forward propagating light field and/or the first backward propagating and the second backward propagating light field are multiplexed, so that the optical transform can be updated without interrupting imaging of the scene.

The multiplexing may comprise wavelength multiplexing and/or temporal multiplexing.

The modified region may be is formed by laser machining

The laser micromachining may be is performed using adaptive optics, which modify wavefront properties of a laser system to counteract the effects of aberration on laser focus

Determining on optical transform may comprise determining a correction matrix to take account of fibre deformation/conformation, wherein determining the optical transform comprises multiplying the correction matrix with an uncorrected transmission matrix.

The uncorrected transmission matrix may be determined by detecting, at the distal end of the fibre, forward propagating light coupled into the optical fibre at the proximal end of the fibre.

The method may further comprise correcting the transmission matrix for temperature determined from a modified region that comprises a fibre Bragg grating.

Also according to the invention in claim 11, there is provided an apparatus for obtaining information for correcting an optical transform imparted by a multimode optical fibre, comprising:

a multimode fibre comprising

a proximal end,
a distal end, and
at least one modified region between the proximal end and distal end, the modified region configured to transmit light toward the proximal end in response to light propagating through the multimode optical fibre from the proximal end to the distal end,

a light source coupled to the proximal end and configured to transmit forward propagating light into the optical fibre;
a detector coupled to the proximal end and configured to detect backward propagating light transmitted from the at least one modified region in response to the forward propagating light.

The apparatus may comprise a processor configured to determine the optical transform from the detected backward propagating light.

The apparatus according to the invention may be configured to perform the method according to the invention, including any of the optional features thereof.

The forward propagating light may be a first forward propagating light field and the backward propagating light is a first backward propagating light field; and

the light source may be configured to transmit a second forward propagating light field for imaging a scene adjacent to the distal end;
the detector may be configured to detect a second backward propagating light field transmitted through the fibre resulting from the second forward propagating light field; and
the apparatus may be configured to use the detected backward propagating light field to correct an image formed using the second backward propagating light field.

The apparatus may be configured to use an optical transform determined from the detected first backward propagating light field to correct the image formed using the second backward propagating light field

There is also provided an endoscope comprising the apparatus of the invention, including any optional features thereof.

BRIEF DESCRIPTION OF THE DRAWINGS

Example embodiments will be described, by way of example only, with reference to the drawings, in which:

Figure 1 shows a method according to an embodiment;
Figure 2 shows a schematic of an apparatus according to an embodiment;
Figure 3 shows a schematic of an apparatus according to an embodiment, employing wavefront control and a multimode fibre for imaging or sensing;
Figure 4 illustrates the reciprocity of the transmission matrix;
Figure 5 and 6 show a modified region comprising a Bragg grating inscribed in a fibre;
Figure 7 and 8 show a modified region comprising a point structure inscribed in a fibre;
Figure 9 and 10 show Bragg gratings, or other structures, inscribed at different radial and azimuthal locations in a fibre.
Figure 11 shows Bragg gratings, or other structures, inscribed at different longitudinal locations in a fibre;
Figure 12 shows Bragg gratings, or other structures, inscribed at different locations along a Fibre for characterising conformational changes;
Figure 13 shows how multiple structures can be inscribed and stacked within a segment of the fibre that may be kept rigid (e.g. by addition of external tubing or by the specimen) at the distal end of a fibre;
Figure 14 shows amplitude multiplexing of the backward propagating light from the modified regions by using Bragg gratings of different length (reflectivity);
Figure 15 shows spectral multiplexing of the backward propagating light from the modified regions by using Bragg gratings of different periodicity;

Figure 16 shows polarisation multiplexing of the backward propagating light from the modified regions by using Bragg gratings of different shapes; and

Figure 17 shows a two-dimensional array of Bragg gratings of varying lengths at different locations for spatially modulating the amplitude of the reflectance signal;

Figure 18 illustrates that two-dimensional arrays of Bragg gratings operating at different wavelengths can be stacked to further multiplex the reflected signal; and

Figure 19 illustrates an embodiment in which the modified region comprises a Bragg near the edge of the core, which may be suitable for serving as a guide star from which the total reflected intensity can be maximised.

DETAILED DESCRIPTION

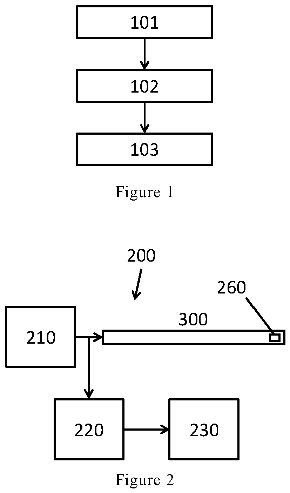

Referring to Figure 1, a method of determining an optical transform imparted by a multimode optical fibre is shown. The multimode fibre comprises a proximal end, a distal end and at least one modified region between the proximal end and distal end. The modified region is configured to transmit backward propagating light toward the proximal end of the fibre in response to forward propagating light transmitted through the optical fibre from the proximal end towards the distal end. The modified region may, for example, comprise a reflective element (such as a fibre Bragg grating) or a fluorescent element. The modified region may be formed by laser machining of the optical fibre, and may be disposed in the fibre core or cladding.

At step 101, forward propagating light is coupled into the proximal end of the MMF. The forward propagating light will reach the modified region in the MMF, and the modified region will cause a backward propagating light signal in response to the forward propagating light (e.g. by fluorescing in response to the forward propagating light, or by reflecting a portion of the forward propagating light).

At step 102, the backward propagating light arising from the interaction of the forward propagating light with the modified region is detected, after it exists from the proximal end of the fibre. A beam splitter may be used to both transmit the forward propagating light into the fibre and to detect the backward propagating light from the modified region. The backward propagating light from the modified region may be imaged, to produce an intensity distribution of the backward propagating light at the proximal end of the fibre.

At step 103, an optical transform TM is determined from the results of detecting the backward propagating light exiting the optical fibre at the proximal end. For example, the intensity distribution of the backward propagating light from the modified region may be used to determine a TM that includes conformation effects, or to determine a correction matrix for correcting a predetermined TM for changes in conformation of the optical fibre.

The detected backward propagating light from the modified region of the fibre can be used to characterise the optical transform imparted by the fibre without access to the distal end. The backward propagating light originates from one or more known positions in the optical fibre (corresponding with one or more modified regions disposed in the optical fibre). The techniques described in the background section can readily be used to characterise the TM in a way that takes into account conformation changes in the fibre to enable imaging using the MMF.

The TM can be used in several ways, which can be grouped into two categories.

The first category encompasses methods in which a distal plane is illuminated with a sequence of non-uniform wide-field illuminations. A distal image can be reconstructed computationally for each image recorded with a proximal detector using the TM. In such an approach, the detector may be used to both determine the TM from the backward propagating light from the modified region, and to obtain the image data from which the distal image is computationally determined using the TM. In some embodiments the determination of the TM may be temporally multiplexed with the acquisition of imaging data (from which the image is determined), for example between each frame, or periodically, depending on how much movement of the MMF is anticipated during imaging (e.g. every 5 imaging frames, or every 10 imaging frames etc). The acquisition of multiple images enables averaging out the nonuniformities in the illuminations due to MMF optical effects. In some embodiments, different light sources and/or different detectors may be used to determine the TM and perform imaging at the same time, using wavelength multiplexing of: the forward propagating light used to interrogate the at least one modified region and the forward propagating light used to image a scene at the distal plane; and/or of the backward propagating light from the one or more modified region and the backward propagating light from the distal plane.

The second category uses the TM, not after data acquisition, but instead to modify the illumination. A wavefront shaping device (such as a spatial light modulator) may be used to modulate the input light field that is used for imaging, so as to shape the light at the distal plane. One implementation of this is to generate a diffraction-limited focal point in the distal plane. This point enables sensing or optical manipulation (e.g. nonlinear photo-polymerisation) in a spatially defined and limited location. The point can also be translated by updating the input field modulation in a fashion equivalent to raster-scanning for realising point-scanning microscopy. Suitable wavefront shaping devices include liquid crystal spatial light modulators and digital micromirror devices.

The methods of characterising the TM described herein are applicable to both categories of using the TM.

Figure 2 illustrates, in simplified form, an apparatus 200 according to an embodiment, comprising a multimode optical fibre 300, light source 210, detector 220 and processor 230.

The multimode optical fibre 300 comprises a modified region 260 near to the distal end thereof (e.g. within 1 cm, or 5mm or 2mm or closer), which may comprise a fibre Bragg grating. In some embodiments the modified region may be closer than 1mm from the distal end (e.g. within 0.5mm or 0.2mm). The modified region 260 may be written using laser machining, which may employ active optics to modify the wavefront properties of the laser to correct for aberration of the laser focus (for example, as described in US2020/166698). In some embodiments there may be a plurality of modified regions, examples of which will be described in more detail below.

The light source 210 is optically coupled to the proximal end of the fibre 300, and transmits forward propagating light into the optical fibre 300. The forward propagating light interacts with the modified region 260, which results in backward propagating light.

The detector 220 is configured to detect the backward propagating light from the interaction of the forward propagating light with the modified region 260, for example by imaging the resulting pattern of light at the proximal end of the fibre 300.

The results from the detector 220 are provided to processor 230 (e.g. in the form of an image of the spatial distribution of light intensity), which determines the TM of the MMF 300 therefrom. Depending on the specific arrangement and nature of the modified region (or regions), any of techniques a) to f) described in the background section may be employed.

In embodiments employing the technique of *Plöschner* there may be one or many Bragg gratings 260, which may be used to characterise conformal changes in the MMF 300. One way to do this is to use the Bragg gratings as strain gauges. For example a distribution of Bragg gratings around the permieter of the fibre can be used to detect bending of the fibre in different directions. As the Bragg grating is strained, the Bragg wavelength defined by the grating spacing changes, which can be detected. Gratings with different Bragg wavelengths can be used, enabling wavelength multiplexed interrogating of the strain gauges. The conformal changes may be used to determine a correction matrix $C_{\lambda, S_{i-1} \rightarrow S_i}$ for updating a predetermined TM (as described in Plöschner).

In some embodiments, the modified region may comprise a single Bragg grating, and the reflected pattern imaged proximally. The intensity distribution may be correlated with a plurality of predetermined intensity distributions in a databank to determine a corresponding TM, following the technique described in *Farahi* (with the Bragg grating serving as a virtual beacon). A single grating may be sufficient to characterise a correction matrix (e.g. for relatively small changes in conformation of the fibre). Multiple gratings may be used to characterise a correction matrix for more significant conformation.

In some embodiments, multiple Bragg gratings can be written at different locations in the fibre. A checkboard reflector following the approach of *Gu* can be generated by employing a similar spatial distribution of Bragg gratings near to the distal tip of the MMF 300, with spatial modulation of the reflectance signal achieved by using Bragg gratings with different lengths (i.e. a different number of periodic variations in refractive index) with longer Bragg gratings providing higher reflectance at the Bragg wavelength.

The multiple Bragg gratings can be probed (by the forward propagating light from the light source 210) at different times, or the light may be wavelength multiplexed. The light incident on the Bragg gratings may be shaped by using wavefront control at the light source 210.

The approach set out in *Gordon* can be followed by employing Bragg gratings with different grating periods, and hence having different Bragg wavelengths. In some embodiments, two-dimensional arrays of Bragg gratings may be stacked (longitudinally near the distal end of the fibre), with each layer in the stack having a different Bragg wavelength. Different layers gratings may be configured with different diattenuation by changing the orientation of the gratings used to define them.

The approach set out in *Li* can be followed by disposing a modified region (Bragg grating or fluorescent centre) to serve as s guide star, from which the TM can be found that optimises constructive interference onto the guide star. The modified region may be disposed at the interface between the core and the cladding, to maximise the size of the isoplanatic patch. Multiple modified regions may be used to tile multiple isoplanatic patches into a single image. Each guide star may partial information of the transmission matrix. The transmission matrix basis can be expressed to make the information obtained from each guide star independent. This is also a good strategy for minimising the number of required guidestars for determining the transmission matrix

Machine learning approaches can also be used. In one example, an image of the back-propagating light from the one or more modified regions is provided to a convolutional neural network (CNN). The CNN may be trained using controlled fibre deformations and corresponding TMs that have been determined with access to both ends of the fibre (for example using methods i) to iii) disclosed in the background section). The CNN may consequently be able to determine a TM directly from a detected image (spatial distribution of intensity) of the back propagating light at the proximal end of the fibre. In some embodiments, the CNN may be provided with image data corresponding with different wavelengths (e.g. reflected from gratings with different period) and with different polarisation states (e.g. reflected from gratings with differently oriented periodic variations in refractive index). The CNN may be configured to determine a correction matrix for correcting a predetermined TM corresponding with a specific conformation of the MMF (e.g. a straight fibre, or a fibre in a neutral starting position of a particular instrument).

Figure 3 shows a further example embodiment of an apparatus 200 according to an embodiment, comprising: light source 210, wavefront shaping device 240, relay lenses 241, aperture 242, beam splitter 250, imaging lens 221, detector 220, coupling lens 231 and multimode optical fibre (MMF) 300.

The MMF 300 comprises a proximal end 310, distal end 320, core, 350, cladding 360 and modified region 260 near to the distal end 320.

The light source 210 is optically coupled to the proximal end 310 of the MMF 300 via the wavefront shaping device 240, relay lenses 241, aperture 242, beam splitter 250 and coupling lens 231. A light beam from the light source 210 is modified by the wavefront shaping device 240, and then coupled into the proximal facet (at the proximal end) of the MMF 300 via the relay lenses 241, aperture 242 and coupling lens 231. The wavefront shaping device 240 may comprise a spatial light modulator, for example employing liquid crystal modulators that are configured to adjust the spatial distribution of phase of the light. The wavefront shaping device 240 may be configured to shape light incident on the modified region, or to perform imaging by shaping light incident on a distal plane (as described above).

The beam splitter 250 is configured to couple forward propagating light from the light sources 210 into the proximal end 310 of the MMF 300, and to coupled backward propagating light emerging from the proximal end 310 of the MMF to the sensor 220, via the imaging lens 221. The detector 220 may comprise a focal plane array, at which an image of the backward propagating light is formed. The detector 220 may be used to detect the backward propagating light from the modified region 260 for characterisation of the TF of the MMF 300, and may also be used to form an image of a scene adjacent to the distal end 320 of the MMF 300 (as described above) using the TF to correct the image. Using the TF to correct/optimise the image may comprise generating a point scanning illuminating light field at the distal scene using the wavefront shaping device 240, and/or may comprise computationally reconstructing an image of the distal scene employing the TF (from one or more images taken from the detector 220).

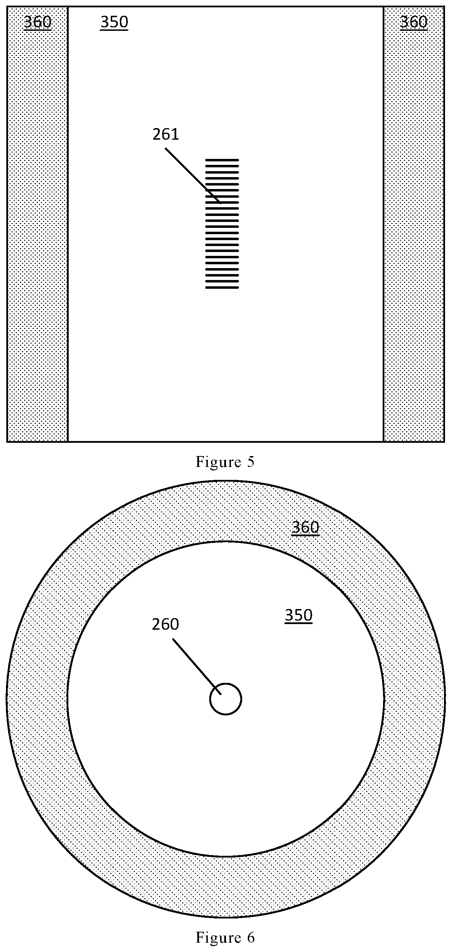

Figures 5 and 6 show an end view and side view respectively of an example of a modified region according to an embodiment comprising a Bragg grating 261 written near the distal end of a MMF. In this example embodiment, the Bragg grating 261 is centrally located, on the axis of the optical fibre core 350.

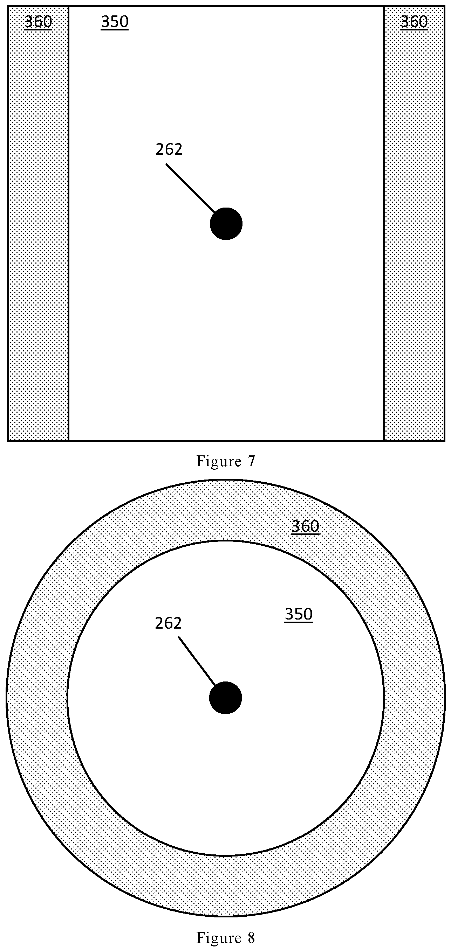

Figures 7 and 8 show an end view and side view respectively of an example of a modified region according to an embodiment comprising a point structure 262, such as a fluorescent centre written near the distal end of a MMF. In this example embodiment, the point structure 262 is centrally located, on the axis of the optical fibre core 350.

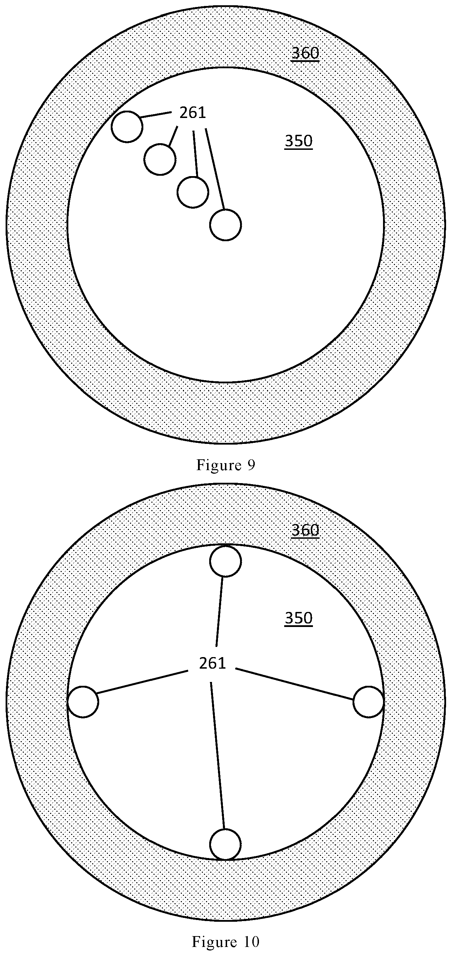

Figure 9 shows an example embodiment in which a plurality of modified regions in the form of Bragg gratings 261 are disposed in the core 350 near the distal end of the MMF. The Bragg gratings 261 are arranged in a one dimensional array along a radius of the fibre core, with different gratings 261 arranged at different radial positions.

Figure 10 shows an example embodiment in which a plurality of modified regions in the form of Bragg gratings 261 are disposed at different circumferential locations at the perimeter of the fibre core 350, near a distal end of the MMF. In the example the gratings 261 are equally spaced around the circumference of the fibre core 350, but this is not essential.

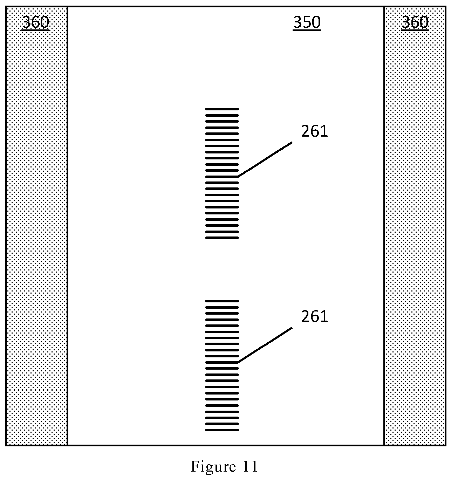

Figure 11 shows a side view of multiple modified regions comprising Bragg gratings 261, disposed on the longitudinal axis of the MMF at different longitudinal positions near to the distal end of the MMF. Although a single Bragg grating at each longitudinal position is shown, there may be more than one Bragg grating at each longitudinal position, and the modified regions may be dispersed both laterally (e.g. in different radial and circumferential positions at a particular longitudinal direction) and longitudinally.

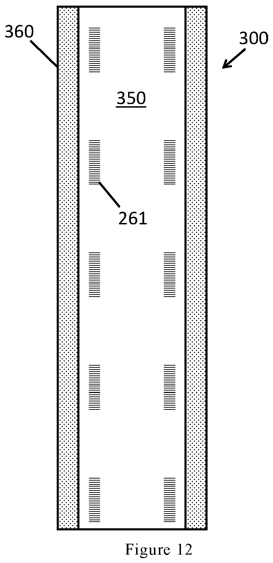

Figure 12 illustrates a side view of multiple modified regions comprising Bragg gratings 261 written near to the edge of the core 350. The Bragg gratings 261 may be evenly distributed along the length of the MMF, with each longitudinal location having a evenly spaced circumferential array of Bragg gratings 261 disposed near to the edge of the core 350 (or in the cladding 360). In such an arrangement each grating is sensitive to bending (with bending at each longitudinal location imparting different strain at different circumferential locations, depending on the direction of bending, which can be detected by a changed in the Bragg wavelength of each grating. Such an arrangement allows conformal changes in the MMF to be sensed. The conformal changes can be used in a calculation as disclosed in *Plöschner* to determine a correction matrix for updating a TM (which may have been predetermined by techniques i) to iii), for example with access to both proximal and distal ends of the MMF.

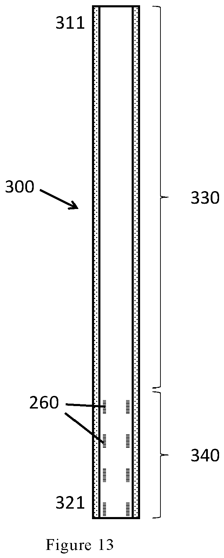

Figure 13 shows an MMF 300 according to an embodiment in which multiple modified regions 260 can be provided in a distal end region 340 of the MMF 300. The multiple modified regions may comprise Bragg gratings, and may be provided at a series of longitudinal positions, with each longitudinal position comprising a plurality of gratings (e.g. as shown in any of Figures 5, 6, 9 and 10). The distal end region 340 of the MMF may be kept substantially free from conformation changes, either by a support structure (such as a rigid support tube) or by the specimen being imaged (e.g. by tissue surrounding the MMF, in the case of invasive endoscopy). The modified regions 260 in the distal end region 340 of the MMF may be used to determine a TM (or a correction matrix) that takes account of conformation changes in the rest of the MMF 330.

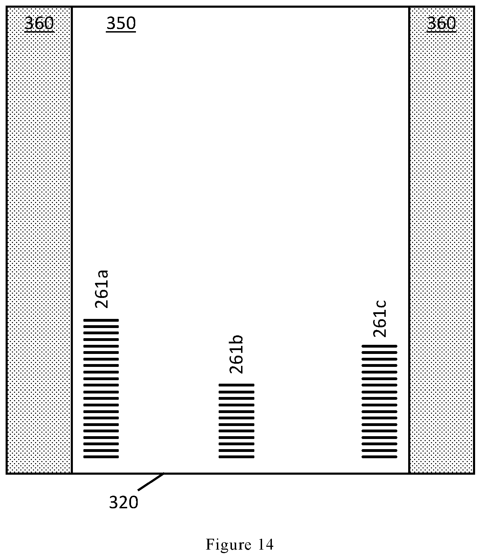

Figure 14 shows a plurality of Bragg gratings 261a, 261b, 261c disposed near a distal end 320 of a MMF in the core 350. Each of the Bragg gratings 261a, 261b, 261c has a different length (number of regions of different refractive index), with the result that each Bragg grating 261a, 261b, 261c has a different reflectance. This enables a checkerboard reflector to be implemented within the fibre that imposes a spatial encoding on the intensity of the reflected light from the modified regions (analogous to the approach of *Gu*).

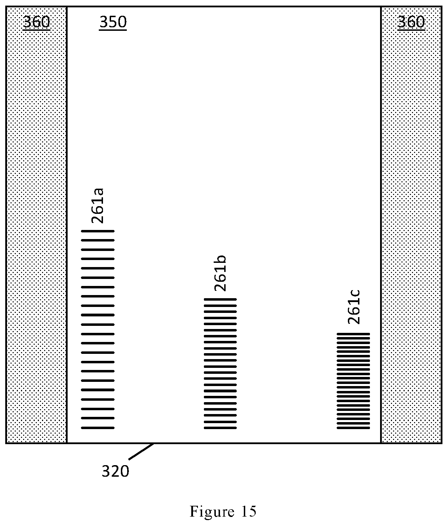

Figure 15 shows a plurality of Bragg gratings 261a, 261b, 261c disposed near a distal end 320 of a MMF in the core 350. Each of the Bragg gratings 261a, 261b, 261c has a different spatial period, with the result that each Bragg grating 261a, 261b, 261c has a different Bragg wavelength (at which it will have maximum reflectance). Different wavelength Bragg gratings may be used to simplify interrogation of multiple modified regions at the same time (by wavelength multiplexing), and also facilitates an approach to determining the TM that is similar to that disclosed in *Gordon*.

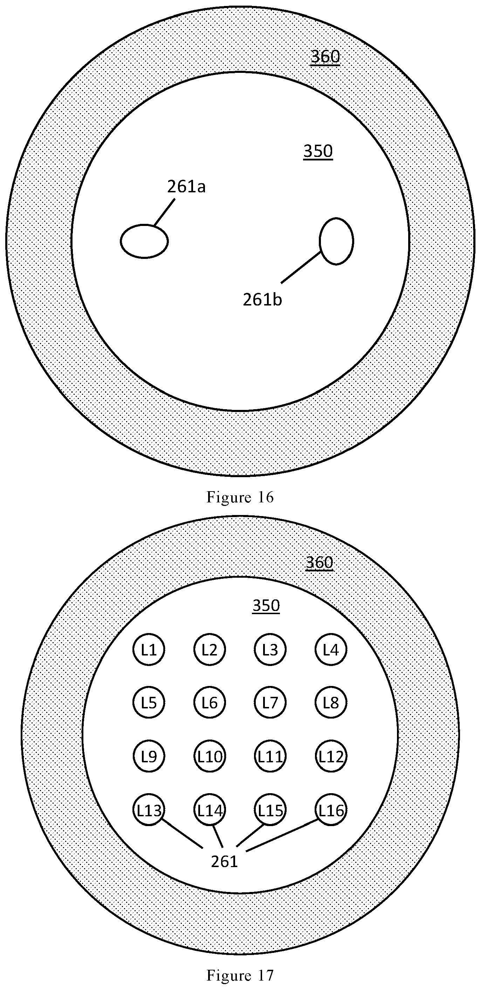

Figure 16 shows a plurality of Bragg gratings 261a, 261b disposed near a distal end 320 of a MMF, in the core 350. Each of the Bragg gratings 261a, 261b has a different orientation of the regions of different refractive index, with the result that each Bragg grating 261a, 261b will have peak reflectance for a different polarisation of light. Polarisation specific Bragg gratings may be used to simplify interrogation of multiple modified regions at the same time (by polarisation multiplexing), and also facilitates an approach to determining the TM that is similar to that disclosed in *Gordon.*

Figure 17 shows a plurality of modified regions L1-L16, disposed in a two dimensional array in the MMF fibre core 350 at a longitudinal location near the distal end of the MMF. The modified regions L1-L16 may comprise Bragg gratings, which may be configured to impose a spatial intensity modulation on the light reflected from the modified regions (e.g. by varying the length of the grating at each location).

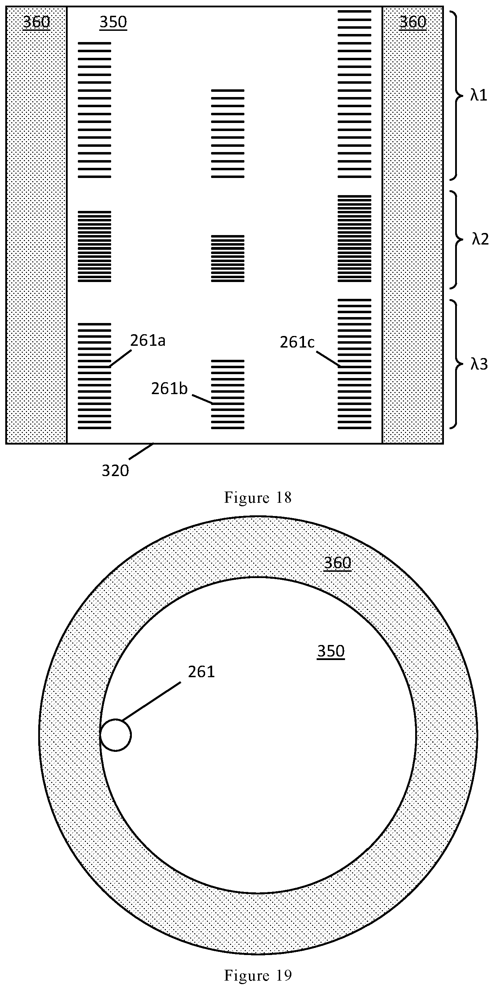

Figure 18 shows a plurality of modified regions 261a, 261b, 261c at each of a plurality of longitudinal positions. The modified regions comprise Bragg gratings, and each longitudinal position comprises Bragg gratings with a particular grating period, so that each layer of Bragg gratings is configured to reflect a specific wavelength of light. Each longitudinal position may be provided with an array of Bragg gratings, as shown in Figure 17. This embodiment may be applicable for determining the TM as disclosed in *Gordon,* with the stacked arrays of Bragg gratings analogous to the stacked metasurfaces in *Gordon.*

Figure 19 shows an embodiment in which a single Bragg grating 261 is provided in the core 350 at the edge of the core 350, near the distal end of the fibre. **In** this embodiment the Bragg grating may serve as a guide star, to determine a TM that enables an isoplanatic patch, as described by *Li.*

Although embodiments with Bragg gratings have been described, some embodiments may employ fluorescent light from the modified regions, rather than reflected light. Since the fluoresced light will have a different wavelength, the sensing of the fluoresced light from the modified regions may be detected at the same time as sensing or imaging using the optical fibre (corrected by the TM determined from the fluoresced light from the modified regions). A secondary focus can be generated on the fluorescent modified region using holography, and temporal multiplexing between multiple fluorescent structures is also possible.

The examples provided in the detailed description are intended to provide examples of the invention, which is only defined by the accompanying claims.

**Claims**

1. A method of determining an optical transform imparted by a multimode optical fibre (300), wherein the multimode optical fibre (300) comprises a proximal end (310), a distal end (320), and at least one modified region (260) between the proximal end (310) and distal end (320), the modified region (260) configured to transmit light toward the proximal end (310) in response to light propagating through the multimode optical fibre (300) from the proximal end (310) to the distal end (320), the method comprising:

   coupling forward propagating light into the proximal end (310) of the multimode optical fibre;
   detecting, at the proximal end (310), backward propagating light transmitted from the at least one modified region (260) in response to the forward propagating light; and
   determining an optical transform from the detected backward propagating light.

2. The method of claim 1, wherein determining the optical transform comprises:

   i) determining a transmission matrix of the multimode optical fibre (300), defining a relationship between an input field at a proximal facet and the resulting output field at a distal facet; and/or
   ii) determining a correction matrix for correcting a predetermined transmission matrix.

3. The method of claim 1 or 2, wherein the at least one modified region comprises:

i) at least one fibre Bragg grating (261, 261a-c); and/or
ii) at least one fluorescent colour centre (262); and/or
iii) one or more modified regions (260) disposed in the fibre core (350) and/or fibre cladding (360).

4. The method of any preceding claim, wherein there are a plurality of modified regions (260), with at least some of the modified regions at different lateral positions and/or at different longitudinal positions between the proximal and distal end.

5. The method of any preceding claim, wherein

i) the at least one modified region (260) comprises a plurality of fibre Bragg gratings (261, 261a-c), and at least some of the fibre Bragg gratings have different: period, reflectivity and/or orientation/polarisation; and/or
ii) the at least one modified region comprises one or more chirped fibre Bragg gratings.

6. The method of any preceding claim, wherein the forward propagating light is a first forward propagating light field and the backward propagating light is a first backward propagating light field, and the method further comprises:

transmitting a second forward propagating light field for sensing/imaging a scene adjacent to the distal end (320); detecting a second backward propagating light field transmitted through the multimode optical fibre (300) resulting from the second forward propagating light field, the method further comprising using the optical transform determined from the first backward propagating light field to correct an image formed using the second backward propagating light field.

7. The method of claim 6, wherein correcting the image comprises computationally reconstructing an image from the results of detecting the second backward propagating light field.

8. The method of claim 6 or 7, wherein the first forward propagating light field and the second forward propagating light field and/or the first backward propagating and the second backward propagating light field are multiplexed, so that the optical transform can be updated without interrupting imaging of the scene.

9. The method of any preceding claim, wherein determining on optical transform comprises determining a correction matrix to take account of fibre deformation, wherein determining the optical transform comprises multiplying the correction matrix with an uncorrected transmission matrix; wherein the uncorrected transmission matrix is optionally determined by detecting, at the distal end (320) of the multimode optical fibre (300), forward propagating light coupled into the multimode optical fibre (300) at the proximal end of the multimode optical fibre (300).

10. The method of any preceding claim, further comprising correcting the transmission matrix for temperature determined from a modified region (260) that comprises a fibre Bragg grating (261, 261a-c).

11. Apparatus (200) for obtaining information for correcting an optical transform imparted by a multimode optical fibre, comprising:

a multimode fibre (300) comprising

a proximal end,
a distal end, and
at least one modified region between the proximal end and distal end, the modified region configured to transmit light toward the proximal end in response to light propagating through the multimode optical fibre (300) from the proximal end (310) to the distal end (320),

a light source (210) coupled to the proximal end (310) and configured to transmit forward propagating light into the optical fibre (300);
a detector (220) coupled to the proximal end (310) and configured to detect backward propagating light transmitted from the at least one modified region (260) in response to the forward propagating light

12. The apparatus (200) of claim 11, further comprising:
a processor (230) configured to determine the optical transform from the detected backward propagating light.

13. The apparatus (200) of claim 13 or 14, wherein the apparatus (200) is configured to perform the method of any preceding claim.

14. An apparatus (200) according to any of claims 11 to 13, wherein the forward propagating light is a first forward propagating light field and the backward propagating light is a first backward propagating light field; and

the light source (210) is configured to transmit a second forward propagating light field for imaging a scene adjacent to the distal end (320);
the detector (220) is configured to detect a second backward propagating light field transmitted through the multimode optical fibre (300) resulting from the second forward propagating light field; and
the apparatus (200) is configured to use the detected backward propagating light field to correct an image formed using the second backward propagating light field.

15. An endoscope comprising the apparatus (200) of any of claims 11 to 14.


**Patentansprüche**

1. Verfahren zum Bestimmen einer optischen Transformation, die durch eine multimodale optische Faser (300) vermittelt wird, wobei die multimodale optische Faser (300) ein proximales Ende (310), ein distales Ende (320) und mindestens eine modifizierte Region (260) zwischen dem proximalen Ende (310) und dem distalen Ende (320) umfasst, wobei die modifizierte Region (260) dazu konfiguriert ist, Licht in Richtung des proximalen Endes (310) als Reaktion darauf zu übertragen, dass sich Licht durch die multimodale optische Faser (300) von dem proximalen Ende (310) zu dem distalen Ende (320) ausbreitet, wobei das Verfahren Folgendes umfasst:

Einkoppeln von sich vorwärts ausbreitendem Licht in das proximale Ende (310) der multimodalen optischen Faser;
Detektieren, an dem proximalen Ende (310), von sich rückwärts ausbreitendem Licht, das von der mindestens einen modifizierten Region (260) als Reaktion auf das sich vorwärts ausbreitende Licht ausbreitet; und
Bestimmen einer optischen Transformation anhand des detektierten, sich rückwärts ausbreitenden Lichts.

2. Verfahren nach Anspruch 1, wobei das Bestimmen der optischen Transformation Folgendes umfasst:

i) Bestimmen einer Übertragungsmatrix der multimodalen optischen Faser (300), Definieren einer Beziehung zwischen einem Eingabefeld an einer proximalen Facette und dem resultierenden Ausgabefeld an einer distalen Facette; und/oder
ii) Bestimmen einer Korrekturmatrix zum Korrigieren einer vorbestimmten Übertragungsmatrix.

3. Verfahren nach Anspruch 1 oder 2, wobei die mindestens eine modifizierte Region Folgendes umfasst:

i) mindestens ein Faser-Bragg-Gitter (261, 261a-c); und/oder
ii) mindestens ein fluoreszierendes Farbzentrum (262); und/oder
iii) eine oder mehrere modifizierte Regionen (260), die in dem Faserkern (350) und/oder dem Fasermantel (360) angeordnet sind.

4. Verfahren nach einem der vorhergehenden Ansprüche, wobei es eine Vielzahl von modifizierten Regionen (260) gibt, wobei sich mindestens einige der modifizierten Regionen an unterschiedlichen Seitenpositionen und/oder an unterschiedlichen Längspositionen zwischen dem proximalen und dem distalen Ende befinden.

5. Verfahren nach einem der vorhergehenden Ansprüche, wobei

i) die mindestens eine modifizierte Region (260) eine Vielzahl von Faser-Bragg-Gittern (261, 261a-c) umfasst und mindestens einige der Faser-Bragg-Gitter eine unterschiedliche Periode, Reflektivität und/oder Ausrichtung/Polarisation aufweisen; und/oder
ii) die mindestens eine modifizierte Region ein oder mehrere gechirpte Faser-Bragg-Gitter umfasst.

6. Verfahren nach einem der vorhergehenden Ansprüche, wobei das sich vorwärts ausbreitende Licht ein erstes, sich vorwärts ausbreitendes Lichtfeld ist und das sich rückwärts ausbreitende Licht ein erstes, sich rückwärts ausbreitendes Lichtfeld ist und wobei das Verfahren ferner Folgendes umfasst:

Übertragen eines zweiten, sich vorwärts ausbreitenden Lichtfeldes zum Erfassen/Bildgeben einer Szene benachbart zu dem distalen Ende (320);
Detektieren eines zweiten, sich rückwärts ausbreitenden Lichtfeldes, das durch die multimodale optische Faser (300) übertragen wird und aus dem zweiten, sich vorwärts ausbreitendem Lichtfeld resultiert, wobei das Verfahren ferner Verwenden der anhand des ersten, sich rückwärts ausbreitenden Lichtfeldes bestimmten optischen Transformation umfasst, um ein unter Verwendung des zweiten, sich rückwärts ausbreitenden Lichtfeldes gebildetes Bild zu korrigieren.

7. Verfahren nach Anspruch 6, wobei das Korrigieren des Bildes rechnerisches Rekonstruieren eines Bildes anhand der Resultate des Detektierens des zweiten, sich rückwärts ausbreitenden Lichtfeldes umfasst.

8. Verfahren nach Anspruch 6 oder 7, wobei das erste, sich vorwärts ausbreitende Lichtfeld und das zweite, sich vorwärts ausbreitende Lichtfeld und/oder das erste, sich rückwärts ausbreitende Lichtfeld und das zweite, sich rückwärts ausbreitende Lichtfeld gemultiplext werden, sodass die optische Transformation aktualisiert werden kann, ohne die Bildgebung der Szene zu unterbrechen.

9. Verfahren nach einem der vorhergehenden Ansprüche, wobei das Bestimmen einer optischen Transformation Bestimmen einer Korrekturmatrix umfasst, um eine Faserverformung zu berücksichtigen, wobei das Bestimmen der optischen Transformation Multiplizieren der Korrekturmatrix mit einer unkorrigierten Übertragungsmatrix umfasst; wobei die unkorrigierte Übertragungsmatrix optional bestimmt wird durch Detektieren, an dem distalen Ende (320) der multimodalen optischen Faser (300), von sich vorwärts ausbreitendem Licht, das in die multimodale optische Faser (300) an dem proximalen Ende der multimodalen optischen Faser (300) eingekoppelt wird.

10. Verfahren nach einem der vorhergehenden Ansprüche, ferner umfassend Korrigieren der Übertragungsmatrix für eine Temperatur, die anhand einer modifizierten Region (260) bestimmt wird, welche ein Faser-Bragg-Gitter (261, 261a-c) umfasst.

11. Vorrichtung (200) zum Erlangen von Informationen zum Korrigieren einer optischen Transformation, die durch eine multimodale optische Faser vermittelt wird, umfassend:

eine Vielzahl von Fasern (300), umfassend
ein proximales Ende,
ein distales Ende und
mindestens eine modifizierte Region zwischen dem proximalen Ende und dem distalen Ende, wobei die modifizierte Region dazu konfiguriert ist, Licht in Richtung des proximalen Endes als Reaktion darauf zu übertragen, dass sich Licht vorwärts durch die multimodale optische Faser (300) von dem proximalen Ende (310) zu dem distalen Ende (320) ausbreitet,
eine Lichtquelle (210), die an das proximale Ende (310) gekoppelt und dazu konfiguriert ist, sich vorwärts ausbreitendes Licht in die optische Faser (300) zu übertragen;
einen Detektor (220), der an das proximale Ende (310) gekoppelt und dazu konfiguriert ist, sich rückwärts ausbreitendes Licht zu detektieren, das von der mindestens einen modifizierten Region (260) als Reaktion auf das sich vorwärts ausbreitende Licht übertragen wird.

12. Vorrichtung (200) nach Anspruch 11, ferner umfassend:
einen Prozessor (230), der dazu konfiguriert ist, die optische Transformation anhand des detektierten, sich rückwärts ausbreitenden Lichts zu bestimmen.

13. Vorrichtung (200) nach Anspruch 13 oder 14, wobei die Vorrichtung (200) dazu konfiguriert ist, das Verfahren nach einem der vorhergehenden Ansprüche durchzuführen.

14. Vorrichtung (200) nach einem der Ansprüche 11 bis 13, wobei das sich vorwärtsausbreitende Licht ein erstes, sich vorwärts ausbreitendes Lichtfeld ist und das sich rückwärts ausbreitende Licht ein erstes, sich rückwärts ausbreitendes Lichtfeld ist; und

wobei die Lichtquelle (210) dazu konfiguriert ist, ein zweites, sich vorwärts ausbreitendes Lichtfeld zum Bildgeben einer Szene benachbart zu dem distalen Ende (320) zu übertragen;

wobei der Detektor (220) dazu konfiguriert ist, ein zweites, sich rückwärts ausbreitendes Lichtfeld zu detektieren, das durch die multimodale optische Faser (300) übertragen wird und aus dem zweiten, sich vorwärts ausbreitenden Lichtfeld resultiert; und wobei die Vorrichtung (200) dazu konfiguriert ist, das detektierte, sich rückwärts ausbreitende Lichtfeld zu verwenden, um ein unter Verwendung des zweiten, sich rückwärts ausbreitenden Lichtfeldes gebildetes Bild zu korrigieren.

15. Endoskop, umfassend die Vorrichtung (200) nach einem der Ansprüche 11 bis 14.

**Revendications**

1. Procédé de détermination d'une transformation optique transmise par une fibre optique multimode (300), dans lequel la fibre optique multimode (300) comprend une extrémité proximale (310), une extrémité distale (320), et au moins une région modifiée (260) entre l'extrémité proximale (310) et l'extrémité distale (320), la région modifiée (260) étant configurée pour transmettre la lumière vers l'extrémité proximale (310) en réponse à la lumière se propageant à travers la fibre optique multimode (300) depuis l'extrémité proximale (310) jusqu'à l'extrémité distale (320), le procédé comprenant :

   le couplage d'une lumière se propageant vers l'avant dans l'extrémité proximale (310) de la fibre optique multimode ;
   la détection, au niveau de l'extrémité proximale (310), d'une lumière se propageant vers l'arrière transmise à partir de l'au moins une région modifiée (260) en réponse à la lumière se propageant vers l'avant ; et
   la détermination d'une transformation optique à partir de la lumière se propageant vers l'arrière détectée.

2. Procédé de la revendication 1, dans lequel la détermination de la transformation optique comprend :

   i) la détermination d'une matrice de transmission de la fibre optique multimode (300), définissant une relation entre un champ d'entrée au niveau d'une facette proximale et le champ de sortie résultant au niveau d'une facette distale ; et/ou
   ii) la détermination d'une matrice de correction pour corriger une matrice de transmission prédéterminée.

3. Procédé de la revendication 1, dans lequel la détermination de la transformation optique comprend :

   i) la détermination d'une matrice de transmission de la fibre optique multimode (300), définissant une relation entre un champ d'entrée au niveau d'une facette proximale et le champ de sortie résultant au niveau d'une facette distale ; et/ou
   ii) la détermination d'une matrice de correction pour corriger une matrice de transmission prédéterminée.

4. Procédé selon l'une quelconque des revendications précédentes, dans lequel il existe une pluralité de régions modifiées (260), dont au moins certaines des régions modifiées se trouvent à différentes positions latérales et/ou à différentes positions longitudinales entre l'extrémité proximale et l'extrémité distale.

5. Procédé selon l'une des revendications précédentes, dans lequel

   i) l'au moins une région modifiée (260) comprend une pluralité de réseaux de Bragg à fibres (261, 261a-c), et au moins certains des réseaux de Bragg à fibres ont une période, une réflectivité et/ou une orientation/polarisation différentes ; et/ou
   ii) l'au moins une région modifiée comprend un ou plusieurs réseaux de Bragg à fibres chirpées.

6. Procédé selon l'une quelconque des revendications précédentes, dans lequel la lumière se propageant vers l'avant est un premier champ lumineux se propageant vers l'avant et la lumière se propageant vers l'arrière est un premier champ lumineux se propageant vers l'arrière, et le procédé comprend en outre :

   la transmission d'un second champ lumineux se propageant vers l'avant pour détecter/imager une scène adjacente à l'extrémité distale (320) ;
   la détection d'un second champ lumineux se propageant vers l'arrière transmis à travers la fibre optique multimode (300) résultant du second champ lumineux se propageant vers l'avant, le procédé comprenant en outre l'utilisation de la transformation optique déterminée à partir du premier champ lumineux se propageant vers

**14**

l'arrière pour corriger une image formée à l'aide du second champ lumineux se propageant vers l'arrière.

7. Procédé selon la revendication 6, dans lequel la correction de l'image comprend la reconstruction informatique d'une image à partir des résultats de la détection du second champ lumineux se propageant vers l'arrière.

8. Procédé selon la revendication 6 ou 7, dans lequel le premier champ lumineux se propageant vers l'avant et le second champ lumineux se propageant vers l'avant et/ou le premier champ lumineux se propageant vers l'arrière et le second champ lumineux se propageant vers l'arrière sont multiplexés, de sorte que la transformation optique peut être mise à jour sans interrompre l'imagerie de la scène.

9. Procédé selon l'une quelconque des revendications précédentes, dans lequel la détermination de la transformation optique comprend la détermination d'une matrice de correction pour tenir compte de la déformation de la fibre, dans lequel la détermination de la transformation optique comprend la multiplication de la matrice de correction avec une matrice de transmission non corrigée ; la matrice de transmission non corrigée est éventuellement déterminée par la détection, à l'extrémité distale (320) de la fibre optique multimode (300), de la lumière se propageant vers l'avant et couplée à la fibre optique multimode (300) à l'extrémité proximale de la fibre optique multimode (300).

10. Procédé selon l'une quelconque des revendications précédentes, comprenant en outre la correction de la matrice de transmission pour la température déterminée à partir d'une région modifiée (260) qui comprend un réseau de Bragg à fibres (261, 261a-c).

11. Appareil (200) permettant d'obtenir des informations pour corriger une transformation optique conférée par une fibre optique multimode, comprenant :

une fibre multimode (300) comprenant
une extrémité proximale,
une extrémité distale, et
au moins une région modifiée entre l'extrémité proximale et l'extrémité distale, la région modifiée configurée pour transmettre la lumière vers l'extrémité proximale en réponse à la lumière se propageant dans la fibre optique multimode (300) de l'extrémité proximale (310) à l'extrémité distale (320),
une source lumineuse (210) couplée à l'extrémité proximale (310) et configurée pour transmettre la lumière se propageant vers l'avant dans la fibre optique (300) ;
un détecteur (220) couplé à l'extrémité proximale (310) et configuré pour détecter la lumière se propageant vers l'arrière transmise par au moins une région modifiée (260) en réponse à la lumière se propageant vers l'avant.

12. Appareil (200) selon la revendication 11, comprenant en outre :
un processeur (230) configuré pour déterminer la transformation optique à partir de la lumière détectée se propageant vers l'arrière.

13. Appareil (200) selon la revendication 13 ou 14, dans lequel l'appareil (200) est configuré pour exécuter le procédé selon l'une quelconque des revendications précédentes.

14. Appareil (200) selon l'une quelconque des revendications 11 à 13, dans lequel la lumière se propageant vers l'avant est un premier champ lumineux se propageant vers l'avant et la lumière se propageant vers l'arrière est un premier champ lumineux se propageant vers l'arrière ; et

la source lumineuse (210) est configurée pour transmettre un deuxième champ lumineux se propageant vers l'avant pour imager une scène adjacente à l'extrémité distale (320) ;
le détecteur (220) est configuré pour détecter un second champ lumineux se propageant vers l'arrière transmis par la fibre optique multimode (300) résultant du second champ lumineux se propageant vers l'avant ; et
l'appareil (200) est configuré pour utiliser le champ lumineux se propageant vers l'arrière détecté pour corriger une image formée à l'aide du second champ lumineux se propageant vers l'arrière.

15. Endoscope comprenant l'appareil (200) selon l'une quelconque des revendications 11 à 14.

101

102

103

Figure 1

200

260

300

210

220

230

Figure 2

Figure 3

$$x \xrightarrow{\quad T_{\lambda,S} \quad} y'$$

$$x' \xleftarrow{\quad T^{\mathsf{T}}_{\lambda,S} \quad} y$$

Figure 4

360 350 360

261

Figure 5

360

350

260

Figure 6

Figure 7

Figure 8

Figure 9

Figure 10

Figure 11

Figure 12

Figure 13

Figure 14

Figure 15

Figure 16

Figure 17

Figure 18

Figure 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10254534 B2
- WO 2020095071 A

- WO 2013144898 A
- US 2020166698 A

**Non-patent literature cited in the description**

- **PAPADOPOULOS, IOANNIS N. et al.** Focusing and scanning light through a multimode optical fiber using digital phase conjugation.. *Optics express*, 2012, vol. 20 (10), 10583-10590
- **DI LEONARDO, ROBERTO ; SILVIO BIANCHI.** Hologram transmission through multi-mode optical fibers.. *Optics express*, 2011, vol. 19 (1), 247-254
- **FARAHI, S. ; ZIEGLER, D. ; PAPADOPOULOS, I. N. ; PSALTIS, D. ; MOSER, C.** Dynamic bending compensation while focusing through a multimode fiber. *Optics Express*, 2013, vol. 21, 22504-22514

- **GU, R. Y ; MAHALATI, R. N. ; KAHN, J. M.** Design of flexible multi-mode fiber endoscope. *Optics Express*, 2015, vol. 23, 26905-26918
- **CHEN, H. ; FONTAINE, N. K. ; RYF, R. ; NEILSON, D. T. ; WINZER, P.** Remote Spatio-Temporal Focusing over Multimode Fiber Enabled by Single-Ended Channel Estimation. *Ieee Journal of Selected Topics in Quantum Electronics*, 2020, 1-1
- **GORDON, G. S. D. et al.** Characterizing Optical Fiber Transmission Matrices Using Metasurface Reflector Stacks for Lensless Imaging without Distal Access. *Phys Rev X*, 2019, vol. 9, 041050
- **LI, S. ; HORSLEY, S. A. R. ; TYC, T. ; CIZMAR, T. ; PHILLIPS, D. B.** Guide-star assisted imaging through multimode optical fibres. *Arxiv*, 2020